# EUROPÄISCHE PATENTSCHRIFT

(11) **EP 1 671 679 B1**
(45) Veröffentlichungstag und Bekanntmachung des Hinweises auf die Patenterteilung: **22.06.2011**
(21) Anmeldenummer: 04106338.9
(22) Anmeldetag: 06.12.2004
(51) Int. Cl.: A61Q 19/00, A61Q 19/08, A61K 8/06, A61K 8/63

(54) **Kosmetische oder dermatologische Zubereitung mit Phytosterolen in Form einer W/O-Emulsion**
Cosmetic or dermatological composition in the form of a water-in-oil emulsion comprising phytosterols
Composition cosmétique ou dermatologique sous la forme d'une émulsion eau-dans-huile comprenant des phytostéroles

(43) Veröffentlichungstag der Anmeldung: 21.06.2006
(73) Patentinhaber: Sebapharma GmbH & Co. KG, 56154 Boppard-Bad Salzig (DE)
(72) Erfinder: Meyer, Thomas, Dipl.-Ing., 56281 Emmelshausen (DE); Gottfreund, Joachim, Dr., 56154 Boppard (DE)
(74) Vertreter: Lippert, Stachow & Partner

(56) Entgegenhaltungen:
- EP-A- 1 000 613
- DE-A1- 3 526 669
- DE-A1- 19 525 822
- DE-C1- 19 652 302
- FR-A- 2 197 959
- US-A- 4 400 295
- US-A1- 2002 098 218
- US-A1- 2004 208 903

## Beschreibung

Die vorliegende Erfindung betrifft kosmetische oder dermatologische Zubereitungen mit Phytosterolen in Form einer W/O-Emulsion. In einer vorteilhaften Ausführungsform betrifft die vorliegende Erfindung W/O-(Wasser-in-Öl)Emulsionen mit Phytosterolen zur Behandlung von Neurodermitis, Dyskeratosen, Narben, Altershaut und Altersflecken, Pruritus, Vitiligo, Sklerodermie und zur Straffung von Bindegewebe.

Die Haut ist das den menschlichen Körper bedeckende oberflächengrößte (ca. 1,5 bis 2 m²) Organ und dient dem physikalischen, chemischen und immunologischen Schutz, der Wärmeregulation und zur Aufnahme von Sinnesreizen. Unter den zuvor genannten vielen Funktionen ist die Barrierefunktion, die das Austrocknen der Haut und somit des gesamten Organismus verhindert, die wohl wichtigste Funktion. Die Epidermis ist als äußerste Schicht der Haut der wohl bedeutendste Teil dieses Gewebes und besteht aus einem mehrschichtigen verhornten Plattenepitel. Die intakte Hornschicht, d.h. eine epidermale Barriere, ist eine wesentliche Voraussetzung für die Schutzfunktion der Haut gegenüber äußeren Einflüssen. Durch Beeinträchtigung der Barrierefunktion kommt es zu einer stärkeren Einwirkung von äußeren Stoffen auf die Haut und den Verlust körpereigener Stoffe, wie Wasser und/oder Elektrolyte aus der Haut, d.h. es führt zu einem transepidermalen Wasserverlust. Es entsteht ein Abnutzungsekzem, das als klinisches Bild Rötung, Trockenheit, Schuppung, Einrisse, Juckreiz der Haut zeigt, und das bei mangelndem Hautschutz in ein Kontaktekzem übergehen kann.

Darüber hinaus können potenzielle Allergene leicht in die Haut eindringen und zur Sensibilisierung führen. Die Entstehung von allergischen Kontaktekzemen kann durch das Abnutzungsekzem gefördert werden.

Bei der Neurodermitis handelt es sich um eine chronische Hauterkrankung aus der Gruppe der Atopien, die durch trockene Haut, Juckreiz und Ekzemneigung gekennzeichnet ist. Sie zeichnet sich durch eine genetisch bedingte überschießende Immunreaktion aus. Die Ursache ist noch weitgehend unbekannt. Wichtig ist daher die Vermeidung individueller Auslösefaktoren und eine konsequente Hautpflege zur Verhinderung von Austrocknen und Juckreiz.

Für die oben genannten Probleme werden zurzeit Präparate angeboten, die die beschädigte Barriere wieder herstellen sollen oder die Haut vor dem Kontakt mit externen Noxen schützen.

Außer ihrer Barrierewirkung gegen externe chemische und physikalische Einflüsse tragen die epidermalen Lipide auch zum Zusammenhalt der Hornschicht bei und haben Einfluss auf die Hautglätte. Die epidermalen Lipide sind über die gesamte Hornschicht verteilt.

Das Zusammenwirken der Lipide mit den anderen feuchtigkeitsbindenden Substanzen in den oberen Hautschichten ist daher für die Regulation der Hautfeuchtigkeit sehr wichtig. Neben den Lipidmischungen und Wasser enthalten Kosmetika in der Regel auch wasserbindende Substanzen.

Medizinische topische Zubereitungen enthalten in der Regel ein oder zwei Medikamente in wirksamer Konzentration. Übliche kosmetische Darreichungsformen sind Emulsionen. Darunter versteht man im Allgemeinen heterogene Systeme aus zwei miteinander nicht oder nur begrenzt mischbaren Flüssigkeiten, die üblicherweise als Phasen bezeichnet werden. Die eine liegt dabei in Form von Tröpfchen vor (disperse oder innere Phase), während die andere Flüssigkeit eine kontinuierliche (kohärente oder innere Phase) bildet.

In der Kosmetik verstärkt sich in den letzten Jahren ein Trend zu natürlichen Verbindungen als Ausgangsmaterial für moderne kosmetische Rezepturen. Eine interessante Gruppe von Verbindungen stellen die Phytosterole dar. Es handelt sich dabei um eine Reihe von Substanzen, die chemisch sehr ähnliche Strukturmerkmale aufweisen, wie Cholesterin, die aber ausschließlich von pflanzlichen Organismen synthetisiert werden können. Phytosterole haben die gleiche Funktion in Pflanzen wie Cholesterin bei Menschen und Tieren, nämlich die Bildung von Zellmembranen. Phytosterole umfassen also pflanzliche Sterole und Stanole sowie deren Fettsäureester.

Unter Phytosterolen werden daher pflanzliche Sterine bezeichnet, wie Ergosterol, Stigmasterol, Sitosterol, Fucosterol, Fungisterol, Campesterol, Brassicasterol und Avenasterol. Sterine sind vom Cholesterin abgeleitete Steroide, die nur in der C₃-Position eine Hydroxygruppe, sonst aber keine funktionellen Gruppen tragen, also formal Alkohole darstellen; daher die Bezeichnung Sterole. Zusätzlich besitzen die 27 bis 30 C-Atome enthaltenden Sterine im allgemeinen eine Doppelbindung in 5/6 Stellung, seltener auch/oder in 7/8, 8/9 und anderen Positionen (z.B. 22/23). In Römpps Chemie-Lexikon (Dr. Otto-Albrecht Neumüller, 8., neubearb. U. erw. Aufl., Stuttgart, Franckh'sche Verlagshandlung, W. Keller & Co., 1987, Bd. 5, Pl-S, Seiten 3976/3977) ist eine Definition der Sterine offenbart. Weiterhin gibt B. Watzl in Ernährungsumschau (2001), Vol. 40, No. 4, Seiten 161 bis 164 (Phytosterine - Charakteristik, Vorkommen, Aufnahme, Stoffwechsel, Wirkungen) eine Übersicht über Phytosterine.

Ein Anwendungsgebiet von Phytosterinen ist die Funktion eines Emulgators. In der DT 26 03 803 A1 wird beispielsweise die Verwendung von Mischungen aus Fettketonen und pflanzlichen Sterinen als Wasser-in-Öl-Emulgatoren beschrieben. Auch die DE 35 26 669 C2 beschreibt ein Emulsionssystem für Emulsionen des Wasser-in-Öl-Typs, das eine Mischung eines Erdalkali- oder metallischen Lanolates und eines Sterols umfasst.

Typische Zoosterine sind Cholesterin, Lanosterin, Spongosterin, Stellasterin.

Sterole stellen also komplexe chemische Stoffe dar, die in tierischen und pflanzlichen Organismen aus Squalen, einem leicht cyclisierbaren Kohlenwasserstoff, biosynthetisiert werden. Phytosterole, also Sterole aus pflanzlichen Rohstoffquellen, werden vor allem in der Kosmetik eingesetzt, da sie einen Repaireffekt an geschädigter oder durch Umwelteinflüsse belasteter Haut zeigen. Eine Übersicht gibt die Arbeit von R. Wachter et al, "Phytosterole - pflanzliche Wirkstoffe in der Kosmetik", Parfümerie und Kosmetik, 75. Jahrgang, Nr. 11/94, S. 755-761.

In der DE 195 37 027 A1 ist eine Kombination aus Sterinen mit Ubichinonen und/oder Plastochinonen beschrieben. Diese Kombination soll eine prophylaktische und restrukturierende Wirkung bei Hautalterung in synergistischer Weise aufweisen.

Weiterhin beschreibt die US 2002/0098218 A1 ein Verfahren zur Regulierung der Säugetierhornhaut, bei dem eine Zusammensetzung auf die Haut aufgebracht wird, die ein oder mehrere Phytostyrole in einem Träger enthält. Als Träger sind insbesondere Wasser-in-Silikon- oder Silikon-in-Wasser-Emulsionen geeignet.

Das Problem beim Arbeiten mit Phytosterolen besteht darin, dass ihre Schmelzpunkte im Bereich von 130°C bis 150°C liegen. Ein solch hoher Schmelzpunkt überfordert die Produktionseinrichtungen, die die Schmelzkessel bis maximal 90°C aufheizen können, bevorzugt zwischen 75°C und 85°C. Unter diesen Bedingungen sind Phytosterole entweder nicht geschmolzen und können deshalb nicht verarbeitet werden oder sie kristallisieren aus, wenn sie vorher im geschmolzenen Zustand vorlagen. Hierauf verweist die DE 195 25 822 A1. Bevorzugt werden technische Fettalkohole mit 12 bis 18 Kohlenstoffatomen wie Kokos-, Palm-, Palmkern- oder Talgfettalkohol. Ein Klarschmelzpunkt zwischen 83°C und 95°C wird erreicht durch Cetearylalkohol in einem Mischungsverhältnis von 40 (Sterol) : 60 (Fettalkohol) oder einem Verhältnis von 50 : 50. Durch Verwendung von Esterquats lässt sich die Lagerstabilität erhöhen, die beispielsweise in einem Temperaturbereich zwischen 35 und 40°C nach kurzer Zeit eine irreversible Tendenz zur Entmischung zeigen und ihre Viskosität einbüßen (vgl. DE 196 52 302 C1).

Es treten also erhebliche Schwierigkeiten bei der Herstellung und Lagerung von Emulsionen, die Phytosterole enthalten, auf. Da die Wirksamkeit der Produkte aber direkt von der Löslichkeit der Phytosterole abhängig ist, konnten bisher nur Produkte mit einem maximalen Gehalt von 1 % an Phytosterolen hergestellt werden (vgl. Wachter et al., vorstehend).

Zur Behandlung von trockener Haut (Neurodermitis), schmerzhaften, rissigen Verhornungsstörungen (Dyskeratosen), Nachbehandlung von Narben, Altershaut und Altersflecken, Hautjucken (Pruritus), Hautflecken (Vitiligo), Sklerodermie und zur Straffung des Bindegewebes wäre es vorteilhaft, W/O-Emulsionen mit einem Gehalt von 2 bis 5 Gew.-% Phytosterolen anzubieten. Über die Schwierigkeiten bei der Herstellung einer O/W-Emulsion berichtet allerdings die DE 101 31 580 A1.

Dieser Weg kann zur Lösung des oben beschriebenen Problems nicht beschritten werden, da ein anderer Emulsionstyp gewählt werden muss.

Die US-A-4,400,295 beschreibt eine Zusammensetzung enthaltend ein Kondensationsprodukt von Fettsäuren und einer basischen Aminosäure und wenigstens eine sekundäre Komponente ausgewählt aus der Gruppe bestehend aus einer Styrolverbindung, einem Fettsäureester mit einem mehrfachen Alkohol, einer Diolkomponente, einem Glycerinphosphorsäureester und einem höheren Alkohol. Die Zusammensetzung kann zur Herstellung von Cremes und Lotionen dienen.

Die US-A-2004/208903 beschreibt eine Hautpflegezusammensetzung enthaltend eine wirksame Menge einer Dialkanoylhydroxyprolinverbindung und einer zweiten Hauptpflegekomponente ausgewählt aus Hexamidinverbindungen, Zuckeraminverbindungen und deren Kombinationen. Die Verbindungen können als Emulsion oder Lösung vorliegen.

Die FR-A-2 197 959 beschreibt eine dispergierende und emulgierende Zusammensetzung mit einem niedrigen Cholesterin-Gehalt, die Wollfettalkohol als Hauptbestandteil enthält, wobei an Stelle von Cholesterin β-Sitosterol vorliegt. In Kombination mit einem Mineralölträger kann eine stabile Wasser-in-Öl-Emulsion gebildet werden, die für die Herstellung kosmetischer Cremes verwendbar ist.

Es ist daher Aufgabe der vorliegenden Erfindung, kosmetische oder dermatologische Zubereitungen in Form von Wasser-in-Öl-Emulsionen mit einem relativ hohen Phytosterolgehalt anzugeben, die eine hohe Lagerstabilität aufweisen, bei Erwärmung beispielsweise auf 35 bis 40°C keine irreversible Tendenz zur Entmischung zeigen und nicht ihre Viskosität einbüßen. Ferner ist es eine Aufgabe der vorliegenden Erfindung, Zubereitungen zur Verfügung zu stellen, welche den Zustand der Haut bei Neurodermitis, Dyskeratosen, Narben, Altershaut und Altersflecken, Pruritus, Vitiligo, Sklerodermie und zur Straffung von Bindegewebe deutlich verbessern.

Diese Aufgabe wird durch die kosmetischen oder dermatologischen Zubereitungen gemäß Anspruch 1 gelöst. Gegebenenfalls kann die erfindungsgemäße Zubereitung in der Kosmetik oder Dermatologie zugelassene übliche Zusatzstoffe enthalten.

Vorteilhafte Ausführungsformen sind in den Unteransprüchen beschrieben.

Die erfindungsgemäßen Zubereitungen liegen als W/O-Emulsionen vor und zeichnen sich durch ein gutes Hautgefühl und durch eine sehr gute Hautpflegeleistung aus. Die erfindungsgemäßen Zubereitungen haben vorzugsweise einen pH-Wert von 5,5 ± 0,5. Dieser pH-Wert kann mittels organischer Säuren wie Milchsäure, Äpfelsäure oder Zitronensäure eingestellt werden.

Überraschenderweise und für den Fachmann nicht voraussehbar war, dass die erfindungsgemäßen Zubereitungen besser als feuchtigkeitsspendende Zubereitungen wirken, besser die Hautglättung fördern, sich durch bessere Pflegewirkung auszeichnen als Stand der Technik Produkte und eine höhere Stabilität gegenüber der Kristallisation der eingesetzten Fettsäuren aufweisen und sich über eine breitere kosmetische Variabilität auszeichnen und über breitere Konsistenz- und Viskositätsbereiche formulieren lassen.

Die Erfindung betrifft daher kosmetische oder dermatologische Zubereitungen mit Phytosterolen in Form von W/O-Emulsionen. Diese Zubereitungen sind insbesondere zur Behandlung von Neurodermitis, Dyskeratosen, Narben, Altershaut und Altersflecken, Pruritus, Vitiligo, Sklerodermie und zur Straffung von Bindegewebe geeignet.

In einer bevorzugten Ausführungsform kann die erfindungsgemäße Zubereitung weiterhin eine oder mehrere Substanzen, ausgewählt aus der Gruppe der Zoosterine, enthalten. Bevorzugt sind hier Cholesterol und/oder Lanosterol.

Die Phytosterole und gegebenenfalls die Zoosterine sind in einer Ölkomponente bzw. einem Ölkörper aufgenommen, enthaltend ein Gemisch, ausgewählt aus wenigstens einem pflanzlichen Öl und/oder einem Pflanzenfett, wenigstens einem Fettalkohol, ausgewählt aus der Gruppe der verzweigten und unverzweigten, gesättigten und ungesättigten Alkylalkohole mit 12 bis 40 Kohlenstoffatomen, und wenigstens einem Fettsäureester, insbesondere C₁₀ bis C₁₂₋Fettsäuremonoestem mit linearen C₄ bis C₈-Alkoholen und C₆ bis C₁₂-Fettalkoholen und mindestens Mineralsäuren.

Der Ölkörper ist in einer Konzentration von 1 bis 20 Gew.-%, bezogen auf das Gesamtgewicht der Formulierung, in der Zubereitung enthalten.

Durch das Aufnehmen der Phytosterole und gegebenenfalls der Zoosterine in dem zuvor definierten Ölkörper wurde überraschenderweise festgestellt, dass der Schmelzpunkt von ca. 140°C auf zwischen 60 bis 70°C herabgesetzt werden konnte, wodurch die Produktionsfähigkeit sichergestellt ist, die bei 75 bis 85°C liegt. Somit können erstmals homogene, lagerstabile, klarschmelzende Mischungen erhalten werden. Gleichzeitig werden die anwendungstechnischen Eigenschaften der Sterole durch den Zusatz der Komponenten des Ölkörpers nicht negativ beeinflusst.

Die weitere Komponente der erfindungsgemäßen Zubereitung ist Wasser. Wasser ist in einer Konzentration von 98 bis 75 Gew.-%, bezogen auf das Gesamtgewicht Zubereitung enthalten.

Die erfindungsgemäßen Zubereitungen können weiterhin einen oder mehrere in der Kosmetik oder der Dermatologie zugelassene Zusatzstoffe enthalten, ausgewählt aus der Gruppe der Emulgatoren, Konsistenzgeber, Feuchthaltemittel, Konservierungsmittel, Parfümierungsmittel, Füllstoffe und Farbstoffe.

Der Emulgator ist ausgewählt aus der Gruppe, bestehend aus Sorbitanolivat, Aluminiumstearat, Magnesiumstearat, Polyglyceryl-6-dioleat, Methylglucosesesquistearat, Dicocoylpentaerythrithyldistearylcitrat (und) Sorbitansesquioleat (und) Bienenwachs (und) Aluminiumstearaten, Polyglyceryl-3-diisostearat, Polyglyceryl-2-dipolyhydroxystearat, Cetearylalkohol (und) Cetearylglucosid, Behenylalkohol, Diisopropyladipat.

In einer besonderen Ausfiihrungsform enthält die erfindungsgemäße Zubereitung 1 bis 5 Gew.-% Glyzerin, bezogen auf das Gesamtgewicht der Zubereitung und gegebenenfalls 0,5 bis 5 Gew.-% Magnesiumsulfat und/oder 0,05 bis 0,3 Gew.-% Coenzym Q10, oder 1 bis 5 Gew.-% Penthenol, bezogen auf das Gesamtgewicht der Zubereitung.

Als Konservierungsmittel wird ein Stoff aus der Gruppe, bestehend aus Phenoxyethanol, Benzylalkohol, Paraben und Sorbinsäure, ausgewählt.

Der pH-Wert der Zubereitung liegt in einem pH-Bereich von 5,0 bis 6,0, d.h. 5,5 ± 0,5. Der pH-Wert wird mittels organischer Säuren, wie z.B. Milchsäure, Äpfelsäure, Zitronensäure oder Gemischen daraus eingestellt.

Als Konsistenzgeber kann die erfindungsgemäße Zubereitung Stoffe enthalten, ausgewählt aus der Gruppe, die aus Cera alba, Citrus aurantium dulcis, Stearyl-Bienenwachs, Behenyl-Bienenwachs, Xanthan-Gummi besteht.

In einer bevorzugten Ausführungsform der Erfindung sind die Phytosterole bzw. gegebenenfalls auch die Zoosterine in einem Ölkörper aufgenommen und zwar in einem Mischungsverhältnis von 30 (Phytosterole und gegebenenfalls Zoosterine): 70 (Ölkomponente). Als Ölkomponente werden solche Stoffe, ausgewählt aus der Gruppe aus Pflanzenöl und/oder Pflanzenfett, gewonnen aus Mandeln, Disteln, Sojabohnen, Sheabutter, Weizenkeimen, Oliven, Erdnüssen, Raps, Jojoba, Persea gratissima, Zea Mays, Oryza sativa, Arachis hypogaea, Prunis dulcis, Triticum vulgare, Simmondsia chinensis, Brassica campestris, Helianthus annuus, Stoffe aus der Gruppe, ausgewählt aus Dicaprylylether, Hexyldecanol und/oder Hexyldecyllaurat, Octyldodecanol, Hexyldecanol, PPG-2 Myristyletherpropionat, hydriertes Rizinusöl im Gemisch mit Diethylhexylcyclohexan, Dicaprylylcarbonat, Orbignya oleifera, Cetearylisononanoat, Cetearylethylhexanoat, Stearinsäure, Hexyllaurat, Glycerylstearat SE, Squalen und/oder Fettsäureester, vorzugsweise Isopropylmyristat; C₁₂ bis C₁₅-Alkylbenzoate, Coco-Caprylat/Caprat, Butyrospermum parkii, eingesetzt.

Erfindungsgemäß besteht der Ölkörper aus 40 bis 60 Teilen Dicaprylylcarbonat, 10 bis 30 Teilen Octyldodecanol, 10 bis 30 Teilen Hexyllaurat und 10 bis 30 Teilen Jojobaöl.

Die Erfindung wird nun anhand eines Beispiels näher beschrieben, ohne dass die Erfindung darauf beschränkt wäre.

Als Basis für die erfindungsgemäßen Versuche mit Phytosterolen wird eine Mischung ausgewählt, die 0,7% Cholesterol, 9,6% Brassicasterol, 34% Campesterol, 0,6% Stigmasterol, 49,7% Sitosterol, 4,9% Avenasterol und 0,5% andere Bestandteile enthält (Generol® R, Cognis Care Chemicals). Der Schmelzpunkt dieser Mischung liegt bei 130 bis 145°C. Schließlich werden alle Versuche bei einem pH-Wert von 5,5 durchgeführt, der als nachteilig anzusehen ist, weil Fettsäuren in einem pH-Bereich von 3,0 bis 8,0 zur Auskristallisation neigen (vgl. DE 191 39 580 A1).

Als besonders vorteilhaft erwies sich bei den erfindungsgemäßen Versuchen ein Mischungsverhältnis von 30 (Phytosterol) : 70 (Ölkörper), der Ölkörper, bestehend aus den Komponenten Dicaprylylcarbonat, Octyldodecanol, Hexyllaurat und Jojobaöl (Simmondsia chinensis). Der Ölkörper enthält 45 Teile Dicaprylylcarbonat, 20 Teile Octyldodecanol, 17,5 Teile Hexyllaurat und 17,5 Teile Jojobaöl. Dadurch wird der Schmelzpunkt von Generol® R von ca. 140°C auf 60 bis 70°C reduziert, wodurch die Produktionsfähigkeit sichergestellt ist, die bevorzugt bei 75 bis 85°C liegt.

Als W/O-Emulgator hat sich besonders vorteilhaft eine Mischung 1 : 1 Polyglyceryl-2-dipolyhydroxystearat und Polyglyceryl-3-diisostearat gezeigt. Beide Emulgatoren wurden bevorzugt mit jeweils 2 Gew.-%, bezogen auf die Gesamtformulierung, eingesetzt.

Die so erhaltene W/O-Emulsion ist in einem Temperaturbereich zwischen -5° C bis +40° C über 4 Monate stabil, d. h. es treten keine Separationen auf.

Die erfindungsgemäßen W/O-Emulsionen zeichneten sich durch ein gutes Hautgefühl und durch eine sehr gute Hautpflegeleistung aus.

Überraschend und für den Fachmann nicht vorhersehbar war, dass die erfindungsgemäßen Zubereitungen besser die Hautglättung förderten, sich durch bessere Pflegewirkung auszeichneten als der Stand der Technik Produkte und eine höhere Stabilität gegenüber der Kristallisation der eingesetzten Fettsäuren aufwiesen.

Die folgende Tabelle 1 führt Lipide auf, die als Einzelsubstanzen oder auch im Gemisch untereinander erfindungsgemäß vorteilhaft sind. Ebenfalls sind die betreffenden Grenzflächenspannungen gegen Wasser in der folgenden Tabelle angegeben.

**Tabelle 1**

| **Handelsname** | **INCI-Bezeichnung** | **(mN/m)** |
|---|---|---|
| Isofol® 14 T | Butyldecanol + Hexyldecanol + Hexyloctanol + Butyloctanol | 27,6 |
| Isofol® 16 | Hexyldecanol | 24,3 |
| Eutanol® G | Octyldodecanol | 24,8 |
| Cetiol® OE | Dicaprylylether | 22,1 |
| Miglyol® 812 | Capryl-/Caprintriglycerid | 21,3 |
| Cegesoft® C24 | Octylpalmitat | 23,1 |
| Estol® 1540 EHC | Octyloctanoat | 30,0 |
| Finsolv® TN | C₁₂₋₁₅ Alkylbenzoat | 21,8 |
| Cetiol® SN | Cetearylisonoanoat | 28,6 |
| Miglyol® 829 | Capryl-/Caprindiglycerylsuccinat | 29,5 |
| Prisorine® 2036 | Octylisostearat | 29,7 |
| Cetiol® LC | Coco-Caprylat/Caprat | 24,8 |
| Cetiol® 868 | Octylstearat | 28,4 |

## Patentansprüche

1. Kosmetische oder dermatologische Zubereitung mit Phytosterolen in Form einer wasser-in-Öl-Emulsion zur Behandlung von Neurodermitis, Dyskeratosen, Narben, Altershaut und Altersflecken, Pruritus, Vitiligo, Sklerodermie und zur Straffung von Bindegewebe, wobei die Zubereitung besteht aus:
a) 1 bis zu 5 Gew.-%, bezogen auf das Gesamtgewicht der Zubereitung, eines oder mehrerer Phytosterole, ausgewählt aus der Gruppe, bestehend aus Ergosterol, Stigmasterol, Sitosterol, Fucosterol, Fungisterol, Campesterol, Brassicasterol und Avenasterol,
b) einen Ölkörper, und
c) Wasser,
**dadurch gekennzeichnet,**
(i) **dass** der Ölkörper aus 40 bis 60 Teilen Dicaprylylcarbonat, 10 bis 30 Teilen Octyldodecanol, 10 bis 30 Teilen Hexyllaurat und 10 bis 30 Teilen Jojobaöl besteht
oder
(ii) **dass** das wenigstens eine Phytosterol und gegebenenfalls das wenigstens eine Zoosterin in dem Ölkörper aufgenommen sind, welcher enthält ein Gemisch, ausgewählt aus wenigstens einem pflanzlichen Öl und/oder einem Pflanzenfett, wenigstens einem Fettalkohol und wenigstens einem Fettsäureester und mindestens Mineralsäuren.

2. Zubereitung gemäß Anspruch 1, **dadurch** g**ekennzeichnet** dass sie weiterhin bis zu 1 Gew.-%, bezogen auf das Gesamtgewicht der Zubereitung, eines oder mehrerer Zoosterine, ausgewählt aus der Gruppe, bestehend aus Cholesterin, Lanosterin, Stellasterin, insbesondere Cholesterol und/oder Lanosterol, enthält.

3. Zubereitung gemäß Anspruch 1 oder 2, **dadurch gekennzeichnet, dass** in dem Ölkörper aufnehmend das wenigstens eine Phytosterol und gegebenenfalls das wenigstens eine Zoosterin der Fettalkohol ausgewählt ist aus der Gruppe der verzweigten und unverzweigten, gesättigten und ungesättigten Alkylalkohole mit 12 bis 40 Kohlenstoffatomen und der wenigstens eine Fettsäureester ein C₁₀ bis C₁₂-Fettsäuremonoester mit linearen C₄ bis C₈-Alkoholen und C₆ bis C₁₂-Fettalkoholen ist.

4. Zubereitung gemäß einem der vorhergehenden Ansprüche 1 bis 3, **dadurch gekennzeichnet, dass** das wenigstens eine Phytosterol und gegebenenfalls das wenigstens eine Zoosterin in dem Ölkörper in einem Mischungsverhältnis von 30 (Phytosterole und gegebenenfalls Zoosterine) : 70 (Ölkörper) aufgenommen sind.

5. Zubereitung gemäß einem der vorhergehenden Ansprüche 1,3 oder 4 **dadurch gekennzeichnet, dass** der Ölkörper aus Stoffen aus der Gruppe ausgewählt ist, bestehend aus Pflanzenöl und/oder Pflanzenfett, gewonnen aus Mandeln, Disteln, Sojabohnen, Sheabutter, Weizenkeimen, Oliven, Erdnüssen, Raps, Jojoba, Persea gratissima, Zea Mays, Oryza sativa, Arachis hypogaea, Prunis dulcis, Triticum vulgare, Simmondsia chinensis, Brassica campestris, Helianthus annuus, Stoffen aus der Gruppe, ausgewählt aus Dicaprylyl Ether, Hexyldecanol und/oder Hexyldezyllaurat, Octyldodecanol, PPG-2 Myristyletherpropionat, hydriertes Rizinusöl im Gemisch mit Diethylhexylcyclohexan, Dicaprylylcarbonat, Orbignya oleifera, Cetearylisononanoat, Cetearylethylhexanoat, Stearinsäure, Hexyllaurat, Glycerylstearat SE, Squalen und/oder Fettsäureester, vorzugsweise Isopropylmyristat; C₁₂ bis C₁₅-Alkylbenzoate, Coco-Caprylat/Caprat, Butyrospermum parkii.

6. Zubereitung gemäß einem der Ansprüche 1 bis 5, **dadurch gekennzeichnet, dass** das Gemisch aus dem Ölkörper und Phytosterolen und gegebenenfalls Zoosterinen einen Schmelzpunkt von zwischen 60 und 70°C aufweist.

7. Zubereitung gemäß einem der Ansprüche 1 bis 6, **dadurch gekennzeichnet, dass** sie weiterhin einen in der Kosmetik oder Dermatologie zugelassenen Zusatzstoff enthält, ausgewählt aus der Gruppe der Emulgatoren, Konsistenzgeber, Feuchthaltemittel, Konservierungsmittel, Parfümierungsmittel, Füllstoffe, Farbstoffe.

8. Zubereitung gemäß Anspruch 7, **dadurch gekennzeichnet, dass** der Emulgator ausgewählt ist aus der Gruppe, bestehend aus Sorbitanolivat, Aluminiumstearat, Magnesiumstearat, Polyglyceryl-6-dioleat, Methylglucosesesquistearat, Dicocoylpentaerythrithyldistearylcitrat (und) Sorbitansesquioleat (und) Bienenwachs (und) Aluminiumstearaten, Polyglyceryl-3diisostearat, Polyglyceryl-2-Dipolyhydroxystearat, Cetearylalkohol (und) Cetearylglucosid, Behenylalkohol, Diisopropyladipat.

9. Zubereitung gemäß Anspruch 7, **dadurch gekennzeichnet, dass** die Zubereitung 1 bis 5 Gew.-% Glyzerin, bezogen auf das Gesamtgewicht der Zubereitung, und gegebenenfalls 0,5 bis 5 Gew.-% Magnesiumsulfat und/oder 0,05 bis 0,3 Gew.-%, Coenzym Q10, oder 1 bis 5 Gew.-% Penthenol, bezogen auf das Gesamtgewicht der Zubereitung, enthält.

10. Zubereitung gemäß Anspruch 7, **dadurch gekennzeichnet, dass** als Konservierungsmittel ein Stoff aus der Gruppe, ausgewählt aus Phenoxyethanol, Benzylalkohol, Paraben und Sorbinsäure in der Zubereitung enthalten ist.

11. Zubereitung gemäß irgendeinem der vorhergehenden Ansprüche 1 bis 10, **dadurch gekennzeichnet, dass** der pH-Wert der Zubereitung in einem Bereich von 5,0 bis 6,0 liegt, wobei insbesondere der pH-Wert mittels organischer Sauren wie Milchsäure, Äpfelsäure oder Zitronensäure eingestellt ist.

12. Zubereitung gemäß irgendeinem der vorhergehenden Ansprüche 1 bis 11, **dadurch gekennzeichnet, dass** sie 0,5 bis 5 Gew.-%, bezogen auf das Gesamtgewicht der Zubereitung, Konsistenzgeber enthält, ausgewählt aus der Gruppe, die aus Cera alba, Citrus aurantium dulcis, Stearyl-Bienenwachs, Behenyl-Bienenwachs, Xanthan-Gummi besteht.

## Claims

1. Cosmetic or dermatological composition in the form of a water-in-oil emulsion comprising phytosterols for the treatment of neurodermitis, dyskeratoses, scars, gerodermia and age marks, pruritus, vitiligo, skleroderma, and for connective tissue tightening, said composition comprising:
a) 1 to 5% by weight, referred to the total weight of the composition, of one or more phytosteroles, selected from the group consisting of ergosterole, stigmasterole, sitosterole, fucosterole, fungisterole, campesterole, brassicasterole and avenasterole,
b) an oil body and
c) water,
**characterized in that**
(i) the oil body consists of 40 to 60 parts of dicaprylylcarbonate, 10 to 30 parts of octyldodecanole, 10 to 30 parts of hexyl-laurate and 10 to 30 parts of jojoba oil, or
(ii) that the at least one phytosterole and possibly the at least one zoosterine are absorbed in said oil body that comprises a mixture selected from at least one vegetable oil and/or vegetable fat, at least one fatty alcohol and at least one fatty acid ester and at least mineral acids.

2. Composition according to claim 1, **characterized in that** it further comprises up to 1% by weight, referred to the total weight of the composition, of one or more zoosterines, selected from the group consisting of cholesterin, lanosterin, stellasterin, particularly cholesterol and/or lanesterol.

3. Composition according to claim 1 or 2 **characterized in that** in a manner absorbed in the oil body, the at least one phytosterole and possibly the at least one zoosterine, the fatty alcohol is selected from the group of branched or non-branched saturated or unsaturated alkylalcohols with 12 to 40 carbon atoms, and the at least one fatty acid ester is a fatty acid mono-ester with linear C₄ to C₈ alcohols and C₆ to C₁₂ fatty acid alcohols.

4. Composition according to any one of the preceding claims 1 to 3, **characterized in that** the at least one phytosterole and possibly the at least one zoosterine are absorbed in the oil body at a mixing ratio of 30 (phytosteroles and possibly zoosterines) : 70 (oil body).

5. Composition according to any one of the preceding claims 1, 3 or 4, **characterized in that** the oil body is selected from substances from the group consisting of vegetable oil and/or vegetable fat, obtained from almonds, thistles, soja beans, shea butter, wheat germs, olives, peanuts, rape, jojoba, persea gratissima, zea mays, oryza sativa, arachis hypogaea, prunis dulcis, triticum vulgare, simmondsia chinensis, brassica campestris, helianthus annuus, substances from the group selected from dicaprylyle ether, hexldecanole and/or hexyldezyl laurate, octydodecanole, PPG-2 myristil ether propionate, hydrated ricinus oil in a mixture with diethylhexylcyclohexane, dicaprylylcarbonate, orbignya oleifera, cetearylisononanoate, cetearylethylhexanoate, stearine acid, hexyl laurate, glyceryl stearate SE, squalene and/or fatty acid ester, preferably isopropylmyristate; C₁₂ to C₁₅ alkylbenzoate, coco caprylate/caprate, butyrospermum parkii.

6. Composition according to any one of the claims 1 to 5, **characterized in that** the mixture of the oil body and the phytosteroles and possibly zoosterines has a melting point between 60 and 70°C.

7. Composition according to any one of the claims 1 to 6, **characterized in that** the same further contains an additive approved in cosmetics and dermatology and selected from the group of emulsifiers, consistency promoters, humectants, preserving agents, perfuming agents, fillers, colorants.

8. Composition according to claim 7, **characterized in that** the emulsifier is selected from the group consisting of sorbitanolivate, aluminum stearate, magnesium stearate, polyglyceryl-6-dioleate, methyl glucose sesquistearate, dicocoylpentaerythrithyldistearylcitrate (and) sorbitane sesquioleate (and) bees wax (and) aluminum stearates, polyglyceryl-3diisosterate, polyglyceryl-2-dipolyhydroxystearate, cetearyl alcohol (and) cetearyl glucoside, behenyl alcohol, diisopropyladipate.

9. Composition according to claim 7, **characterized in that** the composition contains 1 to 5% by weight of glycerine, referred to the total weight of the composition, and possibly 0.5 to 5% by weight of magnesium sulfate and/or 0.05 to 0.3% by weight of coenzyme Q10 or 1 to 5% by weight of penthenol, referred to the total weight of the composition.

10. Composition according to claim 7, **characterized in that** as a preserving agent a substance from the group selected from phenoxyethanole, benzyl alcohol, parabene and sorbic acid is contained in the composition.

11. Composition according to any one of the preceding claims 1 to 10, **characterized in that** the pH of the composition is in a range of 5.0 to 6.0, wherein the pH is particularly adjusted by means of organic acids such as lactic acid, malic acid or citric acid.

12. Composition according to any one of the preceding claims 1 to 11, **characterized in that** the composition contains 0.5 to 5% by weight, referred to the total weight of the composition, of consistency promoters selected from the group consisting of cera alba, citrus aurantium dulcis, stearyle bees wax, behenyle bees wax, xanthane rubber.

## Revendications

1. Composition cosmétique ou dermatologique sous la forme d'une émulsion eau-dans-huile comprenant des phytostéroles et destinée au traitement de la névrodermite, de la dyskératose, des cicatrices, de la vieillisse dans la peau et des tâches de vieillesse, du prurit, du vitiligo, de la sclérodermie et pour le resserrement du tissu conjonctif, la composition consistant en :
a) 1 à 2% en poids, par rapport au poids total de la composition, d'un ou plusieurs de phytostéroles, choisis du groupe comportant l'ergostérole, le stigmastérole, le sitostérole, le fucostérole, le fungistérole, le campestérole, le brassica stérole et l'avenastérole,
b) un corps huileuse et
c) de l'eau,
la composition Ma**caractérisée en ce que**
(i) le corps huileuse comporte 40 à 60 parties de dicaprylyl carbonate, 10 à 30 parties d'octyldodecanole, 10 à 30 parties de hexyl laurate et 10 à 30 parties d'huile de jojoba, ou
(ii) l'au moins un phytostérole et le cas échéant l'au moins un zoostérine sont absorbés dans le corps huileuse qui comporte une mixture choisie parmi l'un au moins d'une huile végétale et/ou d'une graisse végétale, l'un au moins d'un alcool gras et l'un au moins d'un ester d'acide grasse et au moins des acides minéraux.

2. Composition selon la revendication 1, **caractérisée en ce qu'**elle comporte en outre jusqu'à 1% en poids, par rapport au poids total de la composition, un ou plusieurs de zoostérines, choisis du groupe comportant le cholestérine, le lanostérine, le stellastérine, notamment le cholestérole et/ou le lanostérole.

3. Composition selon la revendication 1 ou 2, **caractérisée en ce que**, de manière absorbante dans le corps huileuse, l'au moins un phytostérole et le cas échéant l'au moins un zoostérine, l'alcool gras est choisi du groupe d'alkyl alcools ramifiés et non-ramifiés saturés et non-saturés et comportant 12 à 40 atomes de carbone et **en ce que** l'au moins un ester d'acide gras est un mono ester d'acide gras comportant d'alcools linéaires C₄ à C₈ et d'alcools gras C₆ à C₁₂.

4. Composition selon l'une des revendications précédentes, **caractérisée en ce que** l'au moins un phytostérole et le cas échéant l'au moins un zoostérine sont absorbés dans le corps huileuse à une proportion de mélange de 30 (les phytostéroles et en cas échéant les zoostérines) : 70 (corps huileuse).

5. Composition selon l'une des revendications 1, 3 ou 4, **caractérisée en ce que** le corps huileuse et choisi parmi de substances du groupe comportant l'huile végétale et/ou la graisse végétale, obtenus à partir d'amandes, de chardons, de graines de soja, de beurre de Karité, de germes de blé, d'olives, d'arachides, de colza, de jojoba, de persea gratissima, de blé d'Inde, d'oryza sativa, d'arachis hypogaea, de prunis dulcis, de triticum vulgare, de simmondsia chinensis, de brassica campestris, de helianthus annuus, et choisi parmi de substances du groupe comportant l'ether de dycaprylyle, le hexyl décanole et/ou le laurate de hexyldecyle, l'octyldodecanole, le PPG Ether-2 propionate de myristyle, de l'huile de ricin hydrée en mélange avec le cyclohexane de diéthylehexyle,le carbonate de dicaprylyle, l'orbignya oleifera, le cetearyl isononanoate, le cetearyl éthyle hexanoate, l'acide stéarique, le laurate de hexyle, le stéarate de glycéryle SE, le squalène et/ou l'ester d'acide gras, de préférence le myristate d'isopropyle ; les benzoates d'alkyle C₁₂ à C₁₅, le coco-caprylate/caprate, le butyrospermum parkii.

6. Composition selon l'une des revendications 1 à 5, **caractérisée en ce que** le mélange de corps huileuse et des phytostéroles et le cas échéant des zoostérines à un point de fusion entre 60 et 70°C.

7. Composition selon l'une des revendications 1 à 6, **caractérisée en ce que** la composition comporte un autre additif qui est admis dans l'industrie de cosmétique ou dermatologie, l'additif étant choisi du groupe comportant des émulgateurs, des donneurs de consistance, des humidifiants, des conservateurs, des flaveurants, des charges, des colorants.

8. Composition selon la revendication 7, **caractérisée en ce que** l'émulgateur est choisi du groupe comportant le sorbitan olivate, le stéarate d'aluminium, le stéarate de magnésium, le polyglyceryl-6-dioleate, le methylglucose sesquistéarate, le dicocoylpentaerythrithyldistearylcitrate (et) le sorbitansesquiolate (et) le cire d'abeille (et) les stéarates d'aluminium, le polyglyceryl-3diisostéarate, le polyglyceryl-2-dipolyhydroxystéarate, l'alcool cetearyle (et) le glucoside cetearyle, l'alcool de behenyle, le diisopropyladiapte.

9. Composition selon la revendication 7, **caractérisée en ce que** la composition comporte 1 à 5% en poids de glycérine, par rapport au poids total de la composition, et le cas échéant 0,5 à 5% en poids de sulfate de magnésium et/ou 0,05 à 0,3% en poids de coenzyme Q10 ou 1 à 5% en poids de penthénole, par rapport eu poids total de la composition.

10. Composition selon la revendication 7, **caractérisée en ce que** comme l'agent de conservation la composition comporte une substance du groupe choisi parmi le phenoxy-éthanole, l'alcool de benzyle, le parabène et l'acide sorbique.

11. Composition selon l'une quelconque des revendications 1 à 10, **caractérisée en ce que** le pH de la composition est dans la plage de 5,0 à 6,0, le pH étant réglé notamment au moyen des acides organiques comme l'acide lactique, l'acide malique ou l'acide citrique.

12. Composition selon l'une quelconque des revendications 1 à 11, **caractérisée en ce que** la composition comporte 0,5 à 5% en poids par rapport au poids total de donneurs de consistance choisi du groupe comportant le cera alba, le citrus aurantium dulcis, le cire d'abeille stéaryle, le cire d'abeille behenyle, le caoutchouc de xanthane.
